# EUROPÄISCHE PATENTSCHRIFT

(11) **EP 3 378 524 B1**
(45) Veröffentlichungstag und Bekanntmachung des Hinweises auf die Patenterteilung: **18.10.2023**
(21) Anmeldenummer: 18162469.3
(22) Anmeldetag: 19.03.2018
(51) Int. Cl.: A61M 35/00, A61F 13/40, B65D 47/44, A61F 13/84

(54) **MEDIZINISCHES SYSTEM ZUM AUFTRAGEN EINER FLÜSSIGKEIT, INSBESONDERE EINES ANTISEPTIKUMS, AUF EINEN TUPFER**
MEDICAL SYSTEM FOR APPLYING A LIQUID, IN PARTICULAR AN ANTISEPTIC TO A SWAB
SYSTÈME MÉDICAL DESTINÉ À L'APPLICATION D'UN LIQUIDE, EN PARTICULIER D'UN ANTISEPTIQUE À UN TAMPON

(30) Priorität: 21.03.2017 DE 102017106034
(43) Veröffentlichungstag der Anmeldung: 26.09.2018
(73) Patentinhaber: Bode Chemie GmbH, 22525 Hamburg (DE)
(72) Erfinder: HEGEMANN, Christine, 22453 Hamburg (DE); KUBOTEIT, Stefan, 21220 Seevetal/Maschen (DE); MALLWITZ, Henning, 21244 Buchholz (DE)
(74) Vertreter: Paul Hartmann AG Patents & Licensing

(56) Entgegenhaltungen:
- WO-A2-2006/004407
- US-A- 4 543 096
- US-A1- 2016 332 201
- US-B1- 6 309 124

## Beschreibung

Die vorliegende Erfindung betrifft ein medizinisches System zum Auftragen einer Flüssigkeit, insbesondere eines Antiseptikums, auf einen Tupfer, umfassend einen flexiblen Behälter mit der Flüssigkeit und ein Aufsatzteil für den Behälter. Das Aufsatzteil umfasst einen Greifmechanismus zum Ergreifen und zum Halten des Tupfers. Weiterhin betrifft die vorliegende Erfindung einen Kit umfassend das vorgenannte System und ein Verfahren zum Auftragen einer Flüssigkeit, insbesondere eines Antiseptikums, auf einen Tupfer. In dem Verfahren wird das vorgenannte System eingesetzt.

Vor einem chirurgischen Eingriff wird die Hautoberfläche des Patienten an der Operationsstelle üblicherweise mit einer antiseptischen Flüssigkeit behandelt. Dadurch werden die auf der Hautoberfläche befindlichen Keime abgetötet und das Infektionsrisiko während des chirurgischen Eingriffes minimiert. Es sind verschiedene Möglichkeiten bekannt, wie die Hautoberfläche des Patienten mit der antiseptischen Flüssigkeit behandelt werden kann.

Eine Möglichkeit zur Behandlung der Hautoberfläche mit der antiseptischen Flüssigkeit besteht darin, die antiseptische Flüssigkeit in einer Schale vorzulegen und einen Tupfer in Verbindung mit einer Pinzette oder einer Tupferzange als Greifwerkzeug zu verwenden. Der Tupfer wird in die Schale eingebracht und mit der antiseptischen Flüssigkeit getränkt. Die Hautoberfläche wird dann mit dem feuchten Tupfer abgewischt. Mit der Pinzette beziehungsweise der Zange kann der Tupfer währenddessen gehalten werden. Damit wird ein direkter Kontakt von Patient und medizinischen Personal während der Hautdesinfektion verhindert und das Infektionsrisiko weiter reduziert. Nachteilig an der beschriebenen Vorgehensweise ist, dass die antiseptische Flüssigkeit in der Schale vorgelegt werden muss. Dies ist ein zusätzlicher Arbeitsschritt für das medizinische Personal, da die antiseptische Flüssigkeit aus einem Vorratsbehälter in die Schale umgefüllt werden muss. Dabei wird die antiseptische Flüssigkeit in der Regel auch im Überschuss in die Schale gefüllt, wodurch viel Desinfektionsmittel verbraucht wird.

Im Stand der Technik sind auch Applikatoren bekannt, um die Hautoberfläche mit der antiseptischen Flüssigkeit zu behandeln. Ein solcher Applikator ist zum Beispiel in der Patentanmeldung US2002/0076255A1 beschrieben. Der Applikator umfasst einen Griff mit einem geschlossenen Ende und einem offenen Ende. Eine zerbrechliche Glasampulle mit der antiseptischen Flüssigkeit befindet sich in dem Griff. Weiterhin umfasst der Applikator ein Schaumstoffkissen am offenen Ende des Griffes und ein Mittel zum Öffnen der Glasampulle. Wird dieses Mittel betätigt, fließt die antiseptische Flüssigkeit aus der Ampulle und kann aus dem Applikator über das Schaumstoffkissen austreten. In der Anwendung wird der Applikator an dem Griff gehalten und das Schaumstoffkissen mit der zu desinfizierenden Haut in Kontakt gebracht. Nachteilig an dem vorgenannten Applikator ist sein vergleichsweise komplizierter Aufbau. Nachteilig ist außerdem, dass einzelne Bestandteile des Applikators wie insbesondere die Glasampulle oder das Schaumstoffkissen nicht ausgetauscht werden können.

Die US 4,543,096 A offenbart einen Applikator für Augentropfen. Der Applikator umfasst eine Flasche, an deren Hals mittels eines Ringes zwei Finger befestigt sind, von denen einer beweglich ausgebildet ist. Die Finger sind dafür vorgesehen die Augenlider offen zu halten, wenn die Tropfen in das Auge eingebracht werden.

Die US 2016/0332201 A1 offenbart einen Applikator für die Hautdesinfektion mit einem Behälter und einem Kopfteil. Der Behälter weist einen Verschluss auf. Das Kopfteil weist zwei unbewegliche Flügel auf, an denen ein absorbierendes Material unlösbar befestigt ist. Der Behälter wird geöffnet, indem Behälter und Kopfteil miteinander verbunden und gegeneinander verdreht werden. Ein in dem Behälter enthaltenes Antiseptikum kann dann in das absorbierende Material einströmen, mit dem es auf ein zu desinfizierendes Hautareal verteilt werden kann.

Die WO 2006/004407 A2 beschreibt verschiedene Applikatoren für Vereisungsmittel zur Entfernung von beispielsweise Warzen. Der Applikator kann stab- beziehungsweise schwertförmig mit einem Griff und einem daran befestigten angespitzten porösen Material ausgestaltet sein. Das Vereisungsmittel kann in einer Sprühdose zur Verfügung gestellt werden, um das poröse Material mit dem Vereisungsmittel zu beaufschlagen.

Die US 6,309,124 B1 richtet sich auf einen Applikator für kosmetische Produkte. In einer Kappe des Applikators ist ein ballförmiges poröses Material zentral angeordnet, welches mit dem Kosmetikum imprägniert ist und zum Auftragen des Kosmetikums auf die Haut verwendet wird.

Der vorliegenden Erfindung liegt die Aufgabe zugrunde, die Hautdesinfektion mit einem Applikator für eine antiseptische Flüssigkeit zu verbessern und die Nachteile im Stand der Technik zu überwinden. Insbesondere liegt der vorliegenden Erfindung die Aufgabe zugrunde, einen einfach aufgebauten, einfach anwendbaren sowie vielseitig einsetzbaren Applikator für eine antiseptische Flüssigkeit zur Hautdesinfektion zur Verfügung zu stellen. Die Erfindung löst die Aufgabe mit einem medizinischen System nach Anspruch 1, einem Kit nach Anspruch 13 und einem Verfahren nach Anspruch 14.

Die Erfindung betrifft demnach gemäß einem ersten Aspekt ein medizinisches System zum Auftragen einer Flüssigkeit, insbesondere eines Antiseptikums, auf einen Tupfer nach Anspruch 1.

Gemäß einem zweiten Aspekt betrifft die Erfindung ein Kit nach Anspruch 13.

Ein dritter Aspekt der Erfindung ist ein Verfahren zum Auftragen einer Flüssigkeit, insbesondere eines Antiseptikums, auf einen Tupfer nach Anspruch 14.

Das erfindungsgemäße medizinische System zum Auftragen einer Flüssigkeit auf einen Tupfer umfasst einen flexiblen Behälter mit der Flüssigkeit und mit einem einen Verschluss aufweisenden ersten Auslass. Der Behälter enthält also eine Flüssigkeit, bei der es sich typischerweise um ein Antiseptikum handelt. Der Behälter besitzt weiterhin einen Auslass, welcher im Zusammenhang mit der vorliegenden Erfindung auch als erster Auslass bezeichnet wird. Der Auslass des Behälters ist mit einem Verschluss versehen. Dieser Verschluss kann geöffnet werden. Wird der Verschluss geöffnet, kann die Flüssigkeit aus dem Behälter über den Auslass austreten. Der Austritt der Flüssigkeit kann dadurch bewirkt oder unterstützt werden, indem der Behälter zusammengedrückt wird. Hierfür muss der Behälter flexibel ausgebildet sein. Ein flexibler Behälter kann verformt und zumindest teilweise zusammengedrückt werden ohne dabei zu Bruch zu gehen.

Das erfindungsgemäße medizinische System umfasst weiterhin ein Aufsatzteil für den Behälter mit einem zweiten Auslass. Das Aufsatzteil wird normalerweise separat, also getrennt von dem Behälter im Sinne eines Zubehörteils zur Verfügung gestellt. Das Aufsatzteil kann lösbar an dem Behälter befestigt werden. Wenn der Verschluss des Behälters geöffnet und das Aufsatzteil an dem Behälter befestigt ist, kann die Flüssigkeit den Behälter über den ersten Auslass verlassen und durch das Aufsatzteil zu dem zweiten Auslass fließen. Von dort kann die Flüssigkeit nach außen abgegeben werden.

Das erfindungsgemäße medizinische System ist dadurch gekennzeichnet, dass das Aufsatzteil einen manuellen, zangenartigen Greifmechanismus mit einem ersten Greifarm und einem zweiten Greifarm umfasst. Der Greifmechanismus dient dazu, den Tupfer zu ergreifen und zu halten. Der Greifmechanismus kann mit der Hand bedient werden (daher die Bezeichnung manuell) und ähnelt in seinem prinzipiellen Aufbau und seiner prinzipiellen Wirkweise einer Zange (daher die Bezeichnung zangenartig). Der Greifmechanismus kann wiederholt geöffnet und geschlossen werden, so dass der Tupfer nicht nur ergriffen, sondern auch wieder freigegeben werden kann.

Mit dem erfindungsgemäßen medizinischen System kann ein Applikator für eine antiseptische Flüssigkeit zur Hautdesinfektion erhalten werden. Hierfür wird der Verschluss des Behälters geöffnet und das Aufsatzteil in der Art einer Kappe über dem Auslass des Behälters befestigt. Wird der Behälter dann zusammengedrückt, kann die antiseptische Flüssigkeit auf den mit Hilfe des Greifmechanismus fixierten Tupfer aufgetragen und auf die Haut appliziert werden. Der erfindungsgemäße Applikator umfasst leicht und kostengünstig herstellbare Bauteile, welche in einfacher Weise zu dem erfindungsgemäßen Applikator zusammengefügt werden können. Der erfindungsgemäße Applikator ist somit einfach aufgebaut. Die Anwendung des erfindungsgemäßen Applikators ist intuitiv und einfach, weil sein Greifmechanismus den seit langer Zeit bekannten Tupferzangen ähnelt. Besondere Vorteile des erfindungsgemäßen Applikators sind, dass das Aufsatzteil von dem Behälter wieder gelöst werden kann und dass der Greifmechanismus wiederholt betätigt werden kann. Ein leerer Behälter kann somit gegen einen neuen ausgewechselt werden, wobei das Aufsatzteil weiter verwendet werden kann. Indem der Greifmechanismus wiederholt betätigt werden kann ist es möglich, mehr als einen Tupfer bei der Hautdesinfektion mit dem erfindungsgemäßen Applikator zu verwenden. Durch die Auswechselbarkeit des Behälters und die Verwendbarkeit mit einer Vielzahl von Tupfern ist der erfindungsgemäße Applikator vielseitig sowie flexibel einsetzbar. So ist der erfindungsgemäße Applikator auch zur Desinfektion eines größeren Hautareals geeignet, indem Behälter und Tupfer ausgewechselt werden, wenn der Behälter leer beziehungsweise der Tupfer kontaminiert ist. Der erfindungsgemäße Applikator ist im Unterschied zu dem integralen System der US2002/0076255A1 auch für die wiederholte Desinfektion eines Hautareals mit Tupferwechsel geeignet. Eine solche wiederholte Desinfektion eines Hautareals mit Tupferwechsel ist in der Hautantiseptik üblich.

Wie bereits eingangs erwähnt, handelt es sich bei der Flüssigkeit in dem Behälter typischerweise um eine antiseptische Flüssigkeit (Antiseptikum), insbesondere um eine sterile antiseptische Flüssigkeit. Antiseptika sind im Stand der Technik bekannt und umfassen beispielsweise Alkohole wie Ethanol, 1-Propanol und 2-Propanol, quartäre Ammoniumverbindungen wie Benzalkoniumchlorid, Octenidin, iodhaltige Verbindungen wie Povidon-lod sowie halogenierte Verbindungen wie Chlorhexidin. Vorzugsweise ist die Flüssigkeit in dem Behälter ein Antiseptikum umfassend eine quartäre Ammoniumverbindung, Octenidin oder Chlorhexidin. Besonders bevorzugt handelt es sich bei der Flüssigkeit in dem Behälter um ein Antiseptikum umfassend eine Mischung aus (a) einem Alkohol (zum Beispiel Ethanol, 1-Propanol oder 2-Propanol), und (b) einer quartären Ammoniumverbindung, Octenidin oder Chlorhexidin. Ein solches Antiseptikum tötet Keime schnell (durch die alkoholische Wirkkomponente) und lang anhaltend (durch die Remanenzwirkstoffe (b) wie Octenidin und Chlorhexidin) ab.

Vorzugsweise ist der Behälter im Wesentlichen zylinderförmig (kreiszylinderförmig) ausgestaltet. Die Zylinderform bezieht sich dabei auf den nicht zusammengedrückten Behälter. Ein zylinderförmiger Behälter mit einem dazu passenden Aufsatzteil kann gut in der Hand gehalten werden und schränkt die Bewegungsfreiheit der Greifarme nicht ein. Da der Behälter flexibel sein muss, um zusammengedrückt werden zu können, umfasst das den Behälter bildende Material typischerweise einen Kunststoff, insbesondere einen biegsamen Kunststoff. Vorzugsweise besteht der Behälter aus einem Kunststoff, insbesondere einem biegsamen Kunststoff, wobei der Verschluss des Behälters gegebenenfalls aus einem anderen Material bestehen kann.

Der Auslass des Behälters (erster Auslass) mit dem Verschluss ist typischerweise an einer Oberseite des Behälters positioniert. Die Oberseite eines zylinderförmigen Behälters entspricht geometrisch betrachtet der Deckfläche des Zylinders. Der Auslass des Behälters kann einen Einmalverschluss oder einen Membranverschluss (womit auch ein Septum gemeint ist) aufweisen. Derartige Verschlüsse sind bekannt und werden insbesondere für Ampullen häufig eingesetzt. Ein Einmalverschluss ist einstückig mit dem Behälter ausgebildet und wird zum Öffnen komplett von dem Behälter entfernt. Ein Einmalverschluss ist nicht in der Lage, den Behälter nach dem Öffnen wieder zu verschließen. Die Entfernung des Einmalverschlusses kann auch eine Voraussetzung dafür sein, dass das Aufsatzteil an dem Behälter befestigt werden kann. Ein Membranverschluss wird zum Öffnen üblicherweise nicht komplett von dem Behälter entfernt, sondern wird zum Öffnen lediglich durchstoßen. Entsprechend ist die Entfernung des Membranverschlusses in der Regel auch keine Voraussetzung dafür, dass das Aufsatzteil an dem Behälter befestigt werden kann. Ein Einmalverschluss hat den Vorteil, dass er preiswert und einfach hergestellt werden kann. Ein Membranverschluss hat im Zusammenhang mit der vorliegenden Erfindung den Vorteil, dass die Verschlussöffnung automatisch bei Befestigung des Aufsatzteils an dem Behälter erfolgen kann. Dies wird in einer noch folgenden Ausführungsform der Erfindung weiter erläutert.

Für die vorliegende Erfindung geeignete Behälter sind im Handel verfügbar. Ein Beispiel hierfür ist das Produkt mit der Bezeichnung Hibidil^{®} (Regent Medical Ltd., Großbritannien). Hibidil^{®} enthält ein Desinfektionsmittel auf Basis von Chlorhexidin, welches in im Wesentlichen zylinderförmigen Kunststoffampullen mit Einmalverschluss bereitgestellt wird.

Der Auslass des Aufsatzteils (zweiter Auslass) kann eine einzige Öffnung aufweisen. Ein Aufsatzteil mit einer einzigen Öffnung als Auslass lässt sich leicht herstellen. Alternativ kann der Auslass des Aufsatzteils auch mehrere Öffnungen umfassen. Diese mehreren Öffnungen sind typischerweise kleiner als die zuvor genannte einzige Öffnung und bilden vorzugsweise eine siebartige Anordnung aus. Damit kann die Flüssigkeit besser auf den Tupfer verteilt werden. Die Anzahl der mehreren Öffnungen kann 2 bis 20, bevorzugt 3 bis 15, mehr bevorzugt 3 bis 10 und ganz besonders bevorzugt 3 bis 6 betragen.

Üblicherweise umfasst das Aufsatzteil einen Hohlkörper mit einem ersten offenen Ende und einem zweiten offenen Ende. Der Hohlkörper wird mit seinem ersten offenen Ende an dem Behälter befestigt. Dabei nimmt der Hohlkörper den Auslass des Behälters auf. Das zweite offene Ende bildet den zweiten Auslass und ist dem ersten offenen Ende vorzugsweise gegenüberliegend angeordnet. Der erste und der zweite Greifarm sind außen an dem Hohlkörper befestigt. Vorzugsweise sind der erste und der zweite Greifarm außen an dem Hohlkörper an zwei sich im Wesentlichen gegenüberliegenden Positionen befestigt. Wenn sich die Greifarme gegenüberliegen, kann eine optimale Handhabbarkeit des Greifmechanismus erzielt werden. Der Hohlkörper stellt eine Art Basis des Aufsatzteils dar, an oder in dem die weiteren Komponenten des Aufsatzteils wie beispielsweise der zweite Auslass oder die Greifarme angeordnet sind. Wenn das Aufsatzteil wie vorangehend beschrieben aufgebaut ist, kann die Flüssigkeit in dem Behälter bei geöffnetem Verschluss und am Behälter befestigten Aufsatzteil von dem ersten Auslass zu dem zweiten Auslass fließen. Dabei wird die Flüssigkeit von dem Innenraum des Hohlkörpers von dem ersten Auslass zu dem zweiten Auslass geleitet.

Der Hohlkörper kann beispielsweise im Wesentlichen die Form eines Zylinders (Kreiszylinders) oder eines Kegelstumpfes aufweisen. Eine Grundfläche des Zylinders oder des Kegelstumpfes umfasst das erste offene Ende des Hohlkörpers, welches zur Befestigung des Aufsatzteils am Behälter dient. Eine Deckfläche des Zylinders oder des Kegelstumpfes umfasst das zweite offene Ende des Hohlkörpers, welches den zweiten Auslass bildet. Der erste und der zweite Greifarm sind außen an einer Mantelfläche des Zylinders oder des Kegelstumpfes befestigt. Ein Aufsatzteil mit einem solchen Hohlkörper kann mit dem zylinderförmigen Behälter gut zusammenpassen.

Das Aufsatzteil des erfindungsgemäßen medizinischen Systems umfasst als wesentliche Komponente einen manuellen, zangenartigen Greifmechanismus mit einem ersten Greifarm und einem zweiten Greifarm. Dabei umfasst in einer Variante erfindungsgemäß sowohl der erste als auch der zweite Greifarm einen Kopfabschnitt, einen Gelenkabschnitt und einen Griffabschnitt. Der Kopfabschnitt ist zum Ergreifen und zum Halten des Tupfers vorgesehen ist und kann eine Greifbacke umfassen. Entsprechend ist der Kopfabschnitt vor dem zweiten Auslass angeordnet. Der Gelenkabschnitt ist zur beweglichen Befestigung des Greifarms am Aufsatzteil vorgesehen und ist normalerweise außen an dem Hohlkörper, insbesondere an der Mantelfläche des Hohlkörpers, angeordnet. Der Gelenkabschnitt kann eine scharnierartige Verbindung umfassen, um den Greifarm beweglich an dem Aufsatzteil (beziehungsweise dessen Hohlkörper) zu befestigen. Es ist auch möglich, dass das Aufsatzteil (beziehungsweise dessen Hohlkörper) und der Greifarm einstückig ausgebildet sind, wobei die Verbindung zwischen Aufsatzteil und Greifarm flexibel ist und den Gelenkabschnitt ausbildet. Der Griffabschnitt ist zum Halten und zur Betätigung des Greifmechanismus vorgesehen. Der Griffabschnitt ist behälterseitig angeordnet und weist somit in Richtung des Behälters, wenn Aufsatzteil und Behälter miteinander verbunden sind. Wenn beide Greifarme einen Kopfabschnitt, einen Gelenkabschnitt und einen Griffabschnitt umfassen, kann ein sehr beweglicher und leicht handhabbarer Greifmechanismus erhalten werden.

Alternativ sind in einer weiteren Variante erfindungsgemäß die Greifarme unterschiedlich ausgestaltet. Dabei umfasst der erste Greifarm einen Kopfabschnitt und ist feststehend mit dem Aufsatzteil verbunden, wobei der erste Greifarm an der Verbindungsstelle mit dem Aufsatzteil endet. Der zweite Greifarm umfasst dann einen Kopfabschnitt, einen Gelenkabschnitt und einen Griffabschnitt. Auch in dieser Alternative können die Kopfabschnitte der beiden Greifarme jeweils eine Greifbacke umfassen, um den Tupfer besser ergreifen zu können. Zudem sind die Verbindungsstelle des ersten Greifarms mit dem Aufsatzteil und der Gelenkabschnitt des zweiten Greifarms normalerweise außen an dem Hohlkörper, insbesondere an der Mantelfläche des Hohlkörpers, angeordnet. Wenn ein Greifarm feststehend ausgebildet wird, kann ein einfacher und leicht herstellbarer Greifmechanismus erhalten werden, weil der feststehende Greifarm keinen Gelenkabschnitt und keinen Griffabschnitt umfassen muss.

Weiterhin ist der Greifmechanismus vorzugsweise derart ausgestaltet, dass er in einem unbetätigten Zustand im Wesentlichen geschlossen oder vollständig geschlossen ist und bei Betätigung geöffnet werden kann. Ein solcher Greifmechanismus kann den Tupfer im unbetätigten Zustand selbstständig fixiert halten, womit die Handhabbarkeit des Applikators weiter vereinfacht wird. Insbesondere gestattet ein solcher Greifmechanismus die Benutzung des Applikators mit nur einer Hand, um den Tupfer zu ergreifen und anschließend die Flüssigkeit auf den Tupfer aufzutragen.

Anknüpfend an die vorangegangene bevorzugte Ausführungsform kann das Aufsatzteil mindestens ein federndes Element umfassen, so dass sich der Greifmechanismus selbstständig in eine Ausgangsstellung bewegen kann, wenn sich der Greifmechanismus in einem unbetätigten Zustand befindet. Der erste und der zweite Greifarm, insbesondere der Kopfabschnitt des ersten und des zweiten Greifarms, sind dann derart ausgestaltet, dass der Greifmechanismus in der Ausgangsstellung im Wesentlichen geschlossen oder vollständig geschlossen ist und bei Betätigung geöffnet werden kann. Beispielsweise können die Kopfabschnitte entgegengesetzt bogenförmig ausgestaltet sein und in der Ausgangsstellung eine ovalförmige Anordnung bilden, damit der Greifmechanismus in der Ausgangsstellung im Wesentlichen geschlossen oder vollständig geschlossen ist und bei Betätigung geöffnet werden kann.

Ebenso kann der Greifmechanismus aber auch derart ausgestaltet sein, dass er in einem unbetätigten Zustand geöffnet ist und bei Betätigung geschlossen werden kann. Ein solcher Greifmechanismus hat den Vorteil, dass der Anwender des Applikators selbst bestimmen kann, mit welcher Kraft der Tupfer fixiert wird. Insbesondere kann der Tupfer mit einem solchen Greifmechanismus mit hoher Kraft fixiert werden, um ein unbeabsichtigtes Lösen des Tupfers von dem Applikator zu verhindern. Die Zuverlässigkeit des Applikators wird dadurch erhöht.

In Weiterführung der zuletzt beschriebenen Ausführungsform kann das Aufsatzteil mindestens ein federndes Element umfassen, so dass sich der Greifmechanismus selbstständig in eine Ausgangsstellung bewegen kann, wenn sich der Greifmechanismus in einem unbetätigten Zustand befindet. Der erste und der zweite Greifarm, insbesondere der Kopfabschnitt des ersten und des zweiten Greifarms, sind dann derart ausgestaltet, dass der Greifmechanismus in der Ausgangsstellung geöffnet ist und bei Betätigung geschlossen werden kann. Zum Beispiel können sich die Kopfabschnitte überkreuzen und in der Ausgangsstellung eine x-förmige Anordnung bilden, damit der Greifmechanismus in der Ausgangsstellung geöffnet ist und bei Betätigung geschlossen werden kann.

In einer bevorzugten Ausführungsform der Erfindung ist das medizinische System entlang einer Längsachse im Wesentlichen symmetrisch aufgebaut, wenn das Aufsatzteil an dem Behälter befestigt ist. Der Tupfer ist mit dem Greifmechanismus auf der Längsachse positionierbar. Damit kann der Applikator gut in der Hand gehalten und die Flüssigkeit sehr exakt auf den Tupfer aufgetragen werden.

In einer ganz besonders bevorzugten Ausführungsform der Erfindung umfasst der Behälter oder das Aufsatzteil ein Flatterventil. Flatterventile als solche sind bekannt, zum Beispiel aus der DE19937549B4. Das Flatterventil kann in dem Behälter, insbesondere in dem zylinderförmigen Behälter, angeordnet sein. Vorteilhafter ist es jedoch, wenn das Flatterventil im Aufsatzteil vorhanden ist. Somit können auch im Handel erhältliche Kunststoffampullen, welche in der Regel kein Flatterventil besitzen, für das medizinische System genutzt werden. Ein im Aufsatzteil vorhandenes Flatterventil ist normalerweise in dem Hohlkörper, insbesondere in dem zylinderförmigen oder kegelstumpfförmigen Hohlkörper, angeordnet. Bei einem Applikator mit Flatterventil kann die Flüssigkeit von dem ersten Auslass zu dem zweiten Auslass erst dann fließen, wenn zusätzlich zu dem Behälterverschluss auch das Flatterventil geöffnet ist. Das Flatterventil kann geöffnet werden, indem der Behälter zusammengedrückt wird. Mit dem Flatterventil kann ein vorzeitiges Austreten der Flüssigkeit aus dem Applikator beispielsweise beim Ergreifen des Tupfers vermieden werden. Das Auftragen der Flüssigkeit auf den Tupfer ist bei einem Applikator mit Flatterventil für den Anwender besser kontrollierbar und steuerbar.

Erfindungsgemäß kann das Aufsatzteil lösbar an dem Behälter befestigt werden. Das Befestigen des Aufsatzteils an dem Behälter kann erfolgen, indem das Aufsatzteil dem Behälter aufgeschoben oder aufgesteckt, vorzugsweise einrastend aufgesteckt, wird. Aufsatzteil und Behälter können in diesem Fall also mittels einer steckerartigen Verbindung oder mittels einer Schnappverbindung aneinander lösbar befestigt sein. Um eine besonders sichere und dichte Befestigung des Aufsatzteils an dem Behälter zu erzielen, kann das Aufsatzteil an dem Behälter auch angeschraubt werden. Aufsatzteil und Behälter können hierbei folglich mittels einer schraubartigen Verbindung aneinander lösbar befestigt sein.

Der Behälter kann wie bereits vorstehend beschrieben einen Membranverschluss aufweisen. Hierbei ist es besonders vorteilhaft, wenn das Aufsatzteil ein Mittel zum Durchstoßen eines Membranverschlusses umfasst. Derartige Mittel sind dem Fachmann geläufig. Das Mittel zum Durchstoßen eines Membranverschlusses ist normalerweise in dem Hohlkörper, insbesondere in dem zylinderförmigen oder kegelstumpfförmigen Hohlkörper, angeordnet. Beispielsweise kann das Mittel zum Durchstoßen eines Membranverschlusses einen rohrartigen Dorn umfassen. Flüssigkeit kann den rohrartigen Dorn durchfließen. Der Dorn ist vorzugsweise zentral in dem Hohlkörper angeordnet und weist mit seiner Spitze in Richtung des ersten offenen Endes, welches den mit der Membran verschlossenen Auslass des Behälters aufnimmt. Der Vorteil eines Aufsatzteils mit einem solchen Mittel besteht darin, dass der Membranverschluss des Behälters automatisch geöffnet wird, wenn das Aufsatzteil an dem Behälter befestigt wird. Für den Anwender des medizinischen Systems entfällt dann ein Arbeitsschritt, nämlich die manuelle Öffnung des Verschlusses. Dadurch lässt sich das medizinische System leichter und schneller anwenden.

Vorzugsweise sind der Behälter und das Aufsatzteil Einwegartikel. Der Behälter und das Aufsatzteil werden also nach der Verwendung nicht wieder aufbereitet (beispielsweise erneut befüllt oder sterilisiert), sondern entsorgt. Allgemein können Einwegartikel preisgünstiger hergestellt werden, da sie nur für kurze Zeit verwendet werden und somit geringere Anforderungen hinsichtlich ihrer Haltbarkeit bestehen. Ebenso ist es aber auch denkbar, dass das Aufsatzteil für die Verwendung über einen längeren Zeitraum vorgesehen ist. Das Aufsatzteil muss dann so ausgestaltet sein, dass es wiederholt gereinigt und insbesondere sterilisiert werden kann. Dies kann zum Beispiel erreicht werden, indem für die Herstellung des Aufsatzteils hochwertige und langlebige Kunststoffe eingesetzt werden. Für die Herstellung von Behälter und Aufsatzteil geeignete Kunststoffe sind zum Beispiel Polyethylen (PE), Polyethylenterephthalat (PET) und Polypropylen (PP).

Die Erfindung betrifft gemäß dem zweiten Aspekt einen Kit. Der Kit umfasst ein steriles medizinisches System nach dem ersten Aspekt der Erfindung und einen sterilen Tupfer. Dabei sind alle Komponenten des medizinischen Systems steril, also zum Beispiel auch die Flüssigkeit in dem Behälter. Optional umfasst der Kit zudem eine Gebrauchsanweisung. Mit dem Kit können dem Anwender alle Komponenten zur Verfügung gestellt werden, die er für einen Desinfektionsvorgang benötigt.

Vorzugsweise ist die Flüssigkeit in dem Behälter des Kits ein Antiseptikum umfassend eine quartäre Ammoniumverbindung, Octenidin oder Chlorhexidin. Dabei umfasst der Tupfer vorteilhafterweise synthetische Fasern, beispielsweise aus Polyethylenterephthalat (PET) oder Polypropylen (PP), weil diese die Freigabe der zuvor genannten bevorzugten Antiseptika aus dem Tupfer im Gegensatz zu Baumwollfasern nicht beeinträchtigen. Der synthetische Tupfer kann auch einen Farbstoff umfassen, um die antiseptische Flüssigkeit anzufärben, so dass der mit dem feuchten Tupfer in Kontakt gebrachte Bereich visuell leicht identifiziert werden kann. Besonders bevorzugt umfasst das Antiseptikum in dem Kit zusätzlich zu der quartären Ammoniumverbindung, Octenidin oder Chlorhexidin noch einen Alkohol als antiseptische Wirkkomponente.

Der Kit kann vorteilhafterweise die folgenden weiteren Bestandteile umfassen:
- einen oder mehrere zusätzliche sterile Behälter nach dem ersten Aspekt der Erfindung, und/oder
- ein oder mehrere zusätzliche sterile Aufsatzteile nach dem ersten Aspekt der Erfindung, und/oder
- einen oder mehrere zusätzliche sterile Tupfer.
Insbesondere kann der Kit dann mehr Behälter als Aufsatzteile und optional mehr Tupfer als Aufsatzteile umfassen. Dies trägt der vielseitigen Einsetzbarkeit des erfindungsgemäßen Applikators Rechnung.

Der dritte Aspekt der Erfindung betrifft ein Verfahren zum Auftragen einer Flüssigkeit, insbesondere eines Antiseptikums, auf einen Tupfer.

Das Verfahren umfasst die folgenden Schritte:
i. Bereitstellen eines medizinischen Systems nach dem ersten Aspekt der Erfindung,
ii. Öffnen des Verschlusses des Behälters,
iii. Befestigen des Aufsatzteils an dem Behälter, um einen Applikator auszubilden,
iv. Bereitstellen des Tupfers,
v. Ergreifen und Halten des Tupfers mit dem Greifmechanismus,
vi. optional Ausrichten des Applikators mit dem Tupfer in eine Position, in welcher die Flüssigkeit der Schwerkraft folgend aus dem Behälter auf den Tupfer aufgetragen werden kann,
vii. Auftragen eines Teils der in dem Behälter enthaltenen Flüssigkeit oder der gesamten in dem Behälter enthaltenen Flüssigkeit auf den Tupfer, indem der Behälter einmal oder mehrmals zusammengedrückt wird,
viii. optional Abwischen einer Oberfläche mit dem von dem Applikator gehaltenen Tupfer.

Die Bereitstellung des medizinischen Systems in Schritt i. und die Bereitstellung des Tupfers in Schritt iv. kann mit einem Kit nach dem zweiten Aspekt der Erfindung erfolgen.

In einer bevorzugten Ausführungsform der Erfindung handelt es sich bei der Oberfläche von Schritt viii. um eine Hautoberfläche.

Die Reihenfolge der Verfahrensschritte kann auch von dem oben angegebenen Ablauf abweichen. Ebenso ist es möglich, dass mehrere der Verfahrensschritte gleichzeitig stattfinden. Wenn der Behälter zum Beispiel mit einer Membran verschlossen ist und das Aufsatzteil den zuvor beschriebenen Dorn aufweist, können die Schritte ii. und iii. gleichzeitig stattfinden.

Wenn in Schritt vii. des Verfahrens nur ein Teil der Flüssigkeit auf den Tupfer aufgetragen wird, kann das Verfahren die folgenden weiteren Schritte umfassen:
i. Freigeben des Tupfers,
ii. Bereitstellen eines weiteren Tupfers,
iii. Wiederholen der Schritte v. bis viii. des Verfahrens mit dem weiteren Tupfer.

Zudem kann das Verfahren auch die folgenden weiteren Schritte umfassen:
i. Lösen des Aufsatzteils von dem Behälter,
ii. Bereitstellen eines weiteren Behälters nach dem ersten Aspekt der Erfindung,
iii. Wiederholen der Schritte ii. bis viii. des Verfahrens mit dem weiteren Behälter.

Die Merkmale der bevorzugten Ausführungsformen des ersten Aspektes der Erfindung lassen sich entsprechend auch auf den zweiten und dritten Aspekt der Erfindung übertragen.

### Figurenbeschreibung

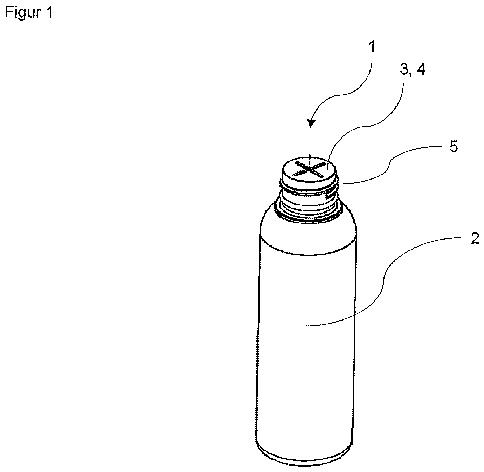

Das mit dem vorliegenden Dokument vorgeschlagene medizinische System wird im Folgenden anhand der Figuren 1 bis 8 beispielhaft veranschaulicht. Dabei können identische Strukturelemente mit denselben Bezugszeichen ausgewiesen sein.

**Figur 1** zeigt einen Behälter **1** des medizinischen Systems in einer Ausführungsform. Der Behälter **1** ist mit einer Flüssigkeit **2** (nicht dargestellt), typischerweise einem Antiseptikum, gefüllt und im Wesentlichen zylinderförmig ausgestaltet. An einer Oberseite des Behälters **1** ist ein Membranverschluss **3** angeordnet, welcher einen Auslass **4** des Behälters **1** überspannt und verschließt. Im oberen Bereich des Behälters **1** befindet sich ein Außengewinde **5,** damit ein Aufsatzteil (nicht dargestellt) angeschraubt werden kann. Der Behälter **1** besteht vorzugsweise aus einem biegsamen Kunststoffmaterial, damit er wie erfindungsgemäß gefordert flexibel ist, um zusammengedrückt werden zu können. Das Erscheinungsbild des Behälters **1** ähnelt einer Ampulle.

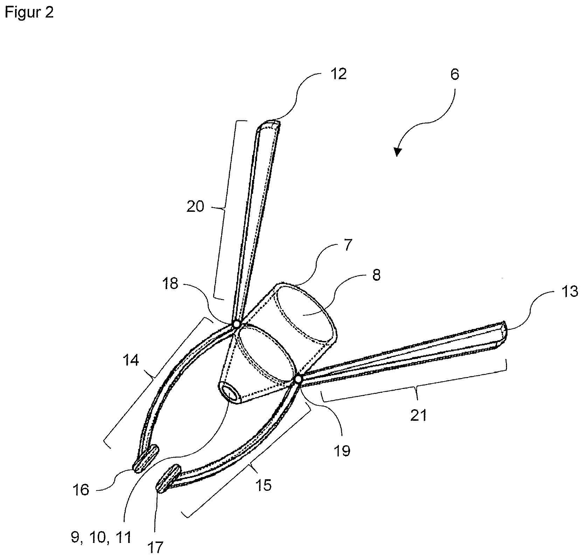

**Figur 2** zeigt ein Aufsatzteil **6** des medizinischen Systems in einer ersten Ausführungsform. Das Aufsatzteil **6** ist passend zu dem Behälter **1** aus **Figur 1** ausgestaltet. Ein Hohlkörper **7** bildet eine Art Basis des Aufsatzteils **6** aus. Der Hohlkörper **7** hat einen zylinderförmigen Abschnitt, welcher in einen kegelstumpfförmigen Abschnitt übergeht. Außen an dem Hohlkörper **7** und vorzugsweise auch innerhalb des Hohlkörpers **7** befinden sich weitere Komponenten des Aufsatzteils **6.** Der Hohlkörper **7** weist ein erstes offenes Ende **8** auf. Mit einem nahe des ersten offenen Endes **8** vorhandenen Innengewinde (nicht dargestellt) kann das Aufsatzteil **6** an der Oberseite des Behälters **1** angeschraubt und somit befestigt werden. Dabei nimmt das erste offene Ende **8** auch den Auslass **4** des Behälters **1** mit dem Membranverschluss **3** auf. Der Hohlkörper **7** weist zudem ein zweites offenes Ende **9** auf, welches dem ersten offenen Ende **8** gegenüberliegt. Das zweite offene Ende **9** bildet einen Auslass **10** des Aufsatzteils **6** aus. Über den Auslass **10** kann Flüssigkeit nach außen abgegeben werden, wenn das Aufsatzteil **6** mit dem Behälter **1** verbunden ist. Bei dem in **Figur 2** gezeigten Aufsatzteil **6** besteht der Auslass **10** aus einer einzigen Öffnung **11.**

An der Mantelfläche des Hohlkörpers **7** sind ein erster Greifarm **12** und ein zweiter Greifarm **13** beweglich befestigt. Die beiden Greifarme **12, 13** bilden einen manuellen, zangenartigen Greifmechanismus, mit dem ein Tupfer ergriffen und gehalten werden kann. Sowohl der erste als auch der zweite Greifarm **12, 13** umfasst einen Kopfabschnitt **14, 15** mit einer Greifbacke **16, 17.** Mit den Kopfabschnitten **14, 15** und insbesondere deren Greifbacken **16, 17** wird der Tupfer ergriffen und gehalten. Die Kopfabschnitte **14, 15** der Greifarme **12, 13** sind entgegengesetzt bogenförmig ausgestaltet. Die Greifarme **12, 13** umfassen weiterhin jeweils einen Gelenkabschnitt **18, 19,** welche die Greifarme **12, 13** beweglich an der Mantelfläche des Hohlkörpers **7** befestigen. Die Gelenkabschnitte **18, 19** können scharnierartig ausgebildet sein. Außerdem umfassen die Greifarme **12, 13** jeweils noch einen Griffabschnitt **20, 21** zum Betätigen des Greifmechanismus. Die Griffabschnitte **20, 21** weisen einen geraden Verlauf auf und stehen dem Hohlkörper **7** in einem Winkel von in etwa 45° ab, wenn der Greifmechanismus geschlossen ist.

Der Greifmechanismus ist in **Figur 2** in einem im Wesentlichen geschlossenen Zustand dargestellt. Vorzugsweise ist das Aufsatzteil **6** so ausgestaltet, dass die dargestellte Position der Greifarme **12, 13** in einem unbetätigten Zustand des Greifmechanismus automatisch vorliegt und gehalten wird. Dies kann zum Beispiel durch federnde Elemente (nicht dargestellt) in den Gelenkabschnitten **18, 19** erreicht werden. Die federnden Elemente drücken die Griffabschnitte **20, 21** von dem Hohlkörper **7** weg, damit der Greifmechanismus im unbetätigten Zustand geschlossen ist.

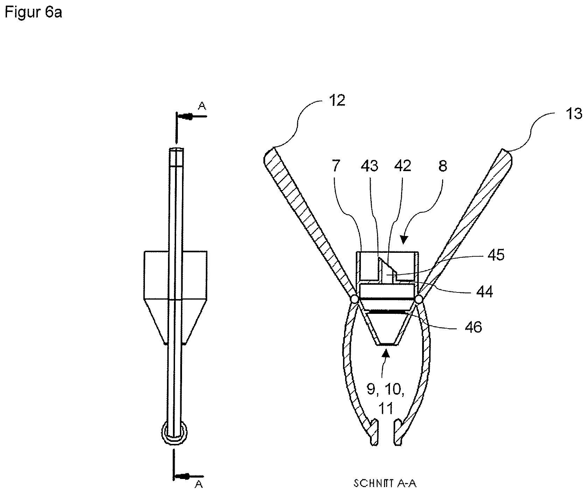

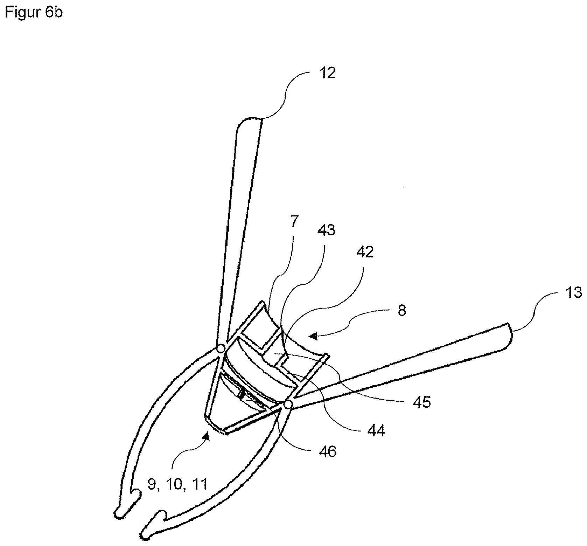

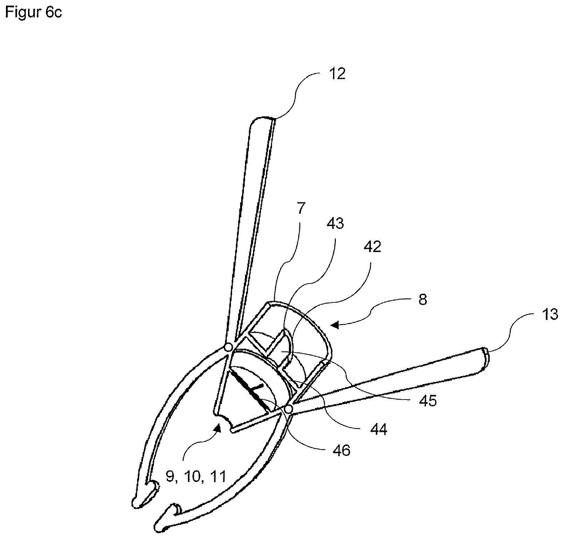

Vorzugsweise sind in dem Hohlkörper **7** noch ein Flatterventil sowie ein Dorn zum Öffnen des Membranverschlusses **3** enthalten. Das Flatterventil und der Dorn sind in **Figur 2** nicht dargestellt, werden aber im Zusammenhang mit **Figur 6** beschrieben.

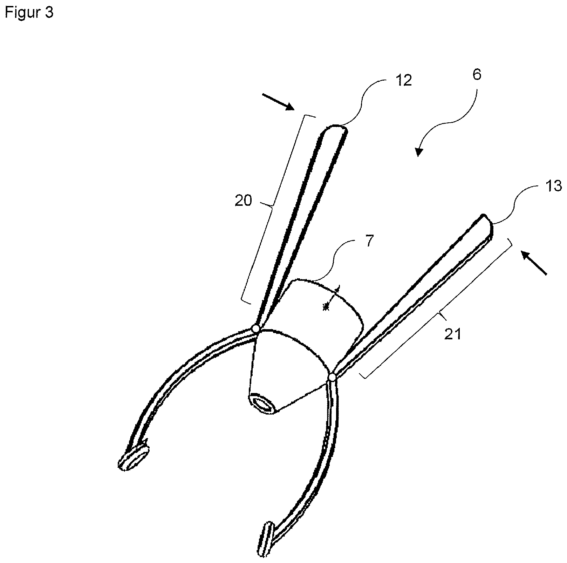

**Figur 3** zeigt das Aufsatzteil **6** aus **Figur 2** mit geöffnetem Greifmechanismus. Der Greifmechanismus wird geöffnet, indem die Greifarme **12, 13** an den Griffabschnitten **20, 21** in Pfeilrichtung bewegt werden.

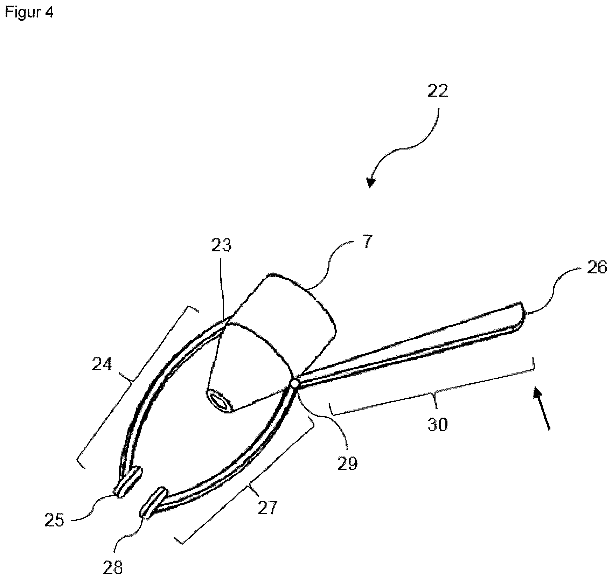

**Figur 4** zeigt ein Aufsatzteil **22** des medizinischen Systems in einer zweiten Ausführungsform. Das Aufsatzteil **22** ist sehr ähnlich wie das zuvor beschriebene Aufsatzteil **6** aufgebaut. Das Aufsatzteil **22** unterscheidet sich von dem Aufsatzteil **6** lediglich im Hinblick auf den Greifmechanismus. Der erste Greifarm **23** weist einen Kopfabschnitt **24** mit einer Greifbacke **25** auf. Der Kopfabschnitt **24** ist feststehend mit dem Hohlkörper **7** verbunden. Der zweite Greifarm **26** weist wie die Greifarme des Aufsatzteils **6** aus **Figur 2** **und** **3** einen Kopfabschnitt **27** mit Greifbacke **28,** einen Gelenkabschnitt **29** und einen Griffabschnitt **30** auf. Wenn der zweite Greifarm **26** in Pfeilrichtung bewegt wird, öffnet sich der Greifmechanismus. Ebenso wie bei dem Aufsatzteil **6** kann der Greifmechanismus des Aufsatzteils **22** in einem unbetätigten Zustand durch beispielsweise ein federndes Element (nicht dargestellt) im Gelenkabschnitt **29** geschlossen gehalten werden. Somit kann ein Tupfer von dem Greifmechanismus selbständig fixiert gehalten werden.

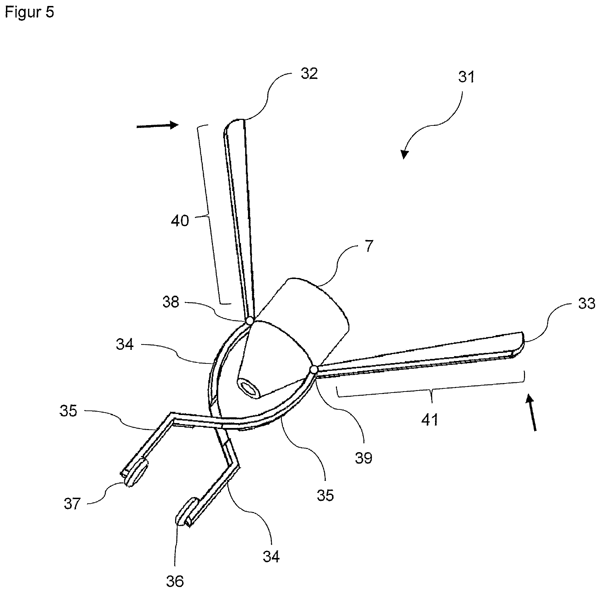

**Figur 5** zeigt ein Aufsatzteil **31** des medizinischen Systems in einer dritten Ausführungsform. Der Hohlkörper **7** ist wie bei den vorangehend beschriebenen Varianten ausgestaltet. Auch die beiden Greifarme **32, 33** weisen grundsätzlich dieselben Strukturelemente wie bei Aufsatzteil **6** auf, nämlich die Kopfabschnitte **34, 35** mit den Greifbacken **36, 37,** die Gelenkabschnitte **38, 39** sowie die Griffabschnitte **40, 41.** Allerdings überkreuzen sich die Kopfabschnitte **34, 35** anstatt entgegengesetzt bogenförmig zu verlaufen. Der Greifmechanismus ist in **Figur 5** in geöffneter Position gezeigt. Diese Position kann durch die im Zusammenhang mit **Figur 2** bereits erläuterten federnden Elemente als eine Ausgangsstellung des Greifmechanismus im unbetätigten Zustand automatisch vorliegen. Erst wenn die Greifarme **32, 33** in Pfeilrichtung bewegt werden, schließt sich der Greifmechanismus und ein Tupfer kann ergriffen und gehalten werden. Zwar kann mit einem solchen Greifmechanismus der Tupfer nicht selbständig fixiert gehalten werden, da sich der Greifmechanismus beim Loslassen der Griffabschnitte **40, 41** automatisch öffnet. Der Tupfer kann jedoch mit hoher Kraft gehalten werden, weil die manuell erzeugten Kräfte auf die Griffabschnitte **40, 41** zum Schließen des Greifmechanismus höher sein können als die von den federnden Elementen erzeugten Kräfte, welche die Griffabschnitte **20, 21** von dem Hohlkörper **7** weg drücken und den Greifmechanismus von Aufsatzteil **6** schließen. Der Vergleich der Aufsatzteile aus **Figur 2** **und** **5** macht jedenfalls deutlich, dass ein veränderter Verlauf der Kopfabschnitte bei ansonsten gleichem Aufbau zu einer Umkehr der Wirkweise des Greifmechanismus führen kann. So ist der Greifmechanismus mit den entgegengesetzt bogenförmigen Kopfabschnitten **14, 15** im unbetätigten Zustand geschlossen und öffnet sich beim Bewegen der Griffabschnitte **20, 21** gemäß **Figur 3****,** wohingegen der Greifmechanismus mit den sich überkreuzenden Kopfabschnitten **34, 35** im unbetätigten Zustand offen ist und sich beim Bewegen der Griffabschnitte **40, 41** in Pfeilrichtung gemäß **Figur 5** schließt.

Ähnlich wie bei dem Aufsatzteil **22** aus **Figur 4** ist es prinzipiell auch hier möglich, den Kopfabschnitt **34** feststehend an dem Hohlkörper **7** auszubilden. Der Gelenkabschnitt **38** und der Griffabschnitt **40** fällt dann weg, was den Greifmechanismus vereinfacht. Dies geht jedoch ebenso wie bei Aufsatzteil **22** mit einer geringeren Beweglichkeit des Greifmechanismus einher.

Die **Figuren 6a bis 6c** zeigen Schnittansichten des Aufsatzteils **6** aus **Figur 2** gemäß einer bevorzugten Ausführungsform (Schnitt entlang der Linie A-A, siehe **Figur 6a****).** Als zusätzliche Komponente ist ein rohrartiger Dorn **42** zu sehen. Der Dorn **42** ist zentral in dem Hohlkörper **7** angeordnet und weist mit seiner Spitze **43** in Richtung des ersten offenen Endes **8,** mit welchem das Aufsatzteil **6** am Behälter **1** befestigt werden kann. In einem unteren Bereich **44** ist der Dorn **42** an die Innenfläche des Hohlkörpers **7** befestigt. Der Dorn **42** bildet aufgrund der rohrartigen Gestalt einen Kanal **45,** welcher Flüssigkeit in Richtung des zweiten offenen Endes **9** leiten kann. Unterhalb des Dornes **42** in Richtung des zweiten offenen Endes **9** ist als zusätzliche Komponente noch ein Flatterventil **46** angeordnet. Das Flatterventil **46** öffnet sich erst, wenn der Behälter zusammengedrückt wird.

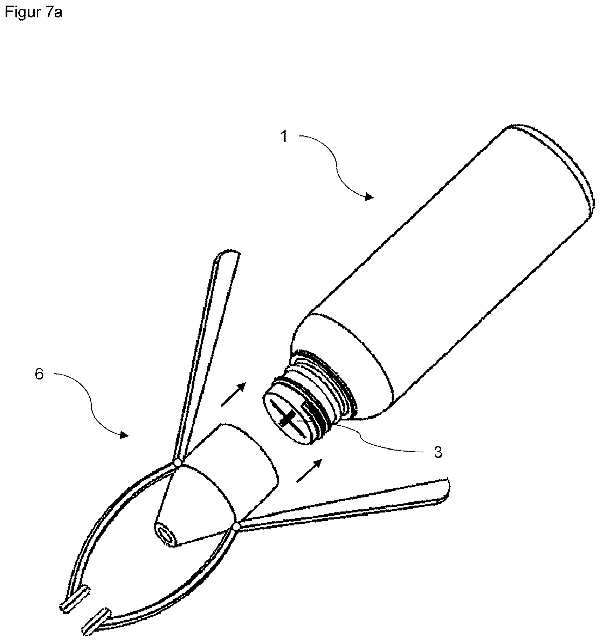

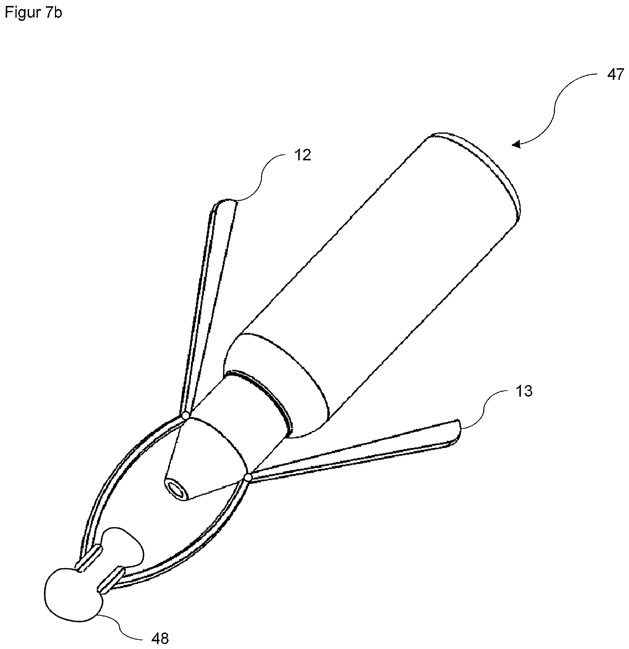

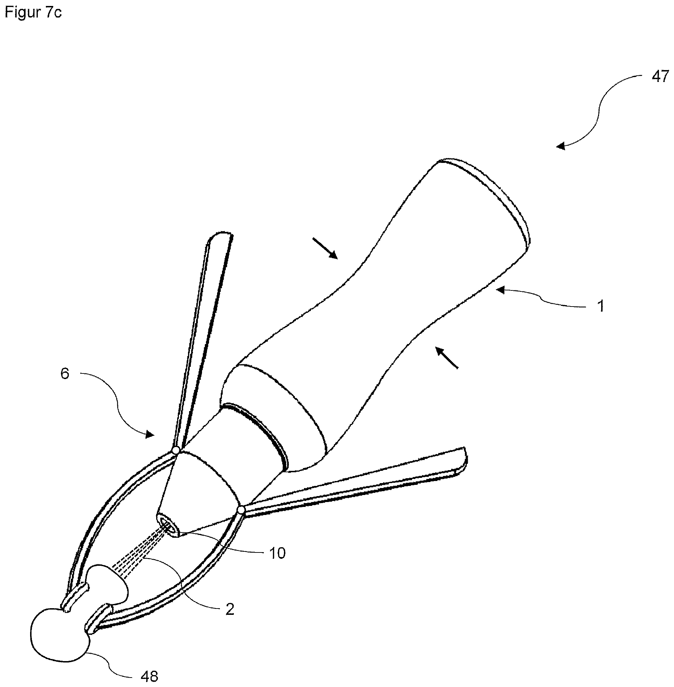

Die **Figuren 7a bis 7c** zeigen beispielhaft die Funktionsweise des medizinischen Systems. **Figur 7a** veranschaulicht, wie der Behälter **1 (****Figur 1**) mit dem Aufsatzteil **6 (****Figuren 2****,** **3** **und** **6****)** verbunden wird. Das Aufsatzteil **6** wird dem Behälter **1** wie dargestellt angeschraubt. Dabei öffnet der in dem Aufsatzteil vorhandene Dorn **42** (nicht dargestellt) automatisch den Membranverschluss **3** des Behälters **1.** Das Flatterventil **46** (nicht dargestellt) verhindert, dass Flüssigkeit aus dem Aufsatzteil **6** austritt, solange der Behälter **1** nicht zusammengedrückt wird. Durch das Befestigen des Aufsatzteils **6** an dem Behälter **1** wird der in **Figur 7b** gezeigte Applikator **47** erhalten. Der Applikator **47** kann wie in **Figur 7b** dargestellt einen Tupfer **48** mit dem Greifmechanismus (**12, 13**) halten. **Figur 7c** schließlich zeigt wie die Flüssigkeit **2,** typischerweise ein Antiseptikum, durch Zusammendrücken des Behälters **1** auf den Tupfer **48** aufgetragen werden kann. Durch das Zusammendrücken des Behälters **1** öffnet sich das Flatterventil **46** (nicht dargestellt) und die Flüssigkeit **2** kann den Applikator **47** über den zweiten Auslass **10** verlassen, um von dort der Schwerkraft folgend auf den Tupfer **48** zu fallen. Mit dem feuchten, von dem Applikator **47** gehaltenen Tupfer **48** kann dann eine Hautoberfläche abgewischt und desinfiziert werden.

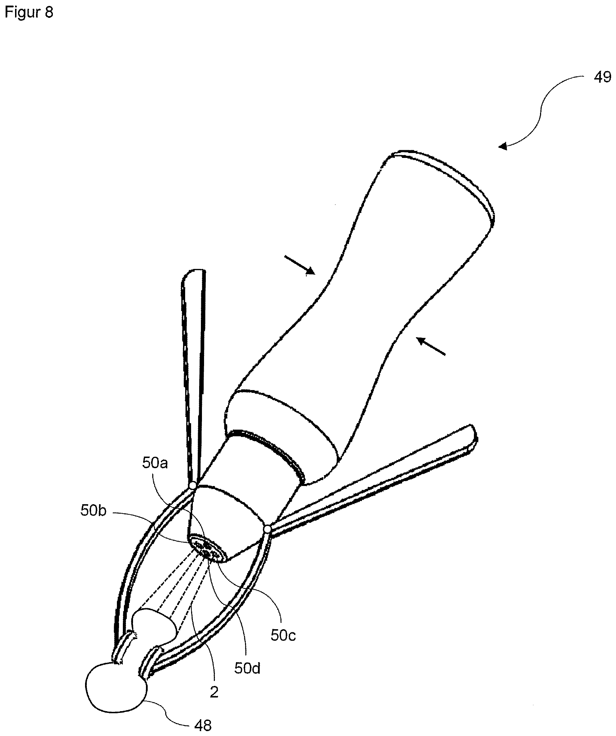

**Figur 8** zeigt einen Applikator **49,** mit dem die Flüssigkeit **2** wie bei **Figur 7c** auf den Tupfer **48** aufgetragen wird. Der Applikator **49** unterscheidet sich von dem Applikator **47** dadurch, dass der zweite Auslass mehrere Öffnungen **50a, 50b, 50c, 50d** umfasst. Die Anzahl der Öffnungen (in Figur 8 sind vier Öffnungen eingezeichnet) ist rein beispielhaft zu verstehen. Insbesondere bilden die mehreren Öffnungen **50a, 50b, 50c, 50d** eine siebartige Anordnung aus. Dadurch kann die Flüssigkeit **2** wie in Figur **8** angedeutet besser verteilt und besser dosiert auf den Tupfer **48** aufgetragen werden.

## Patentansprüche

1. Medizinisches System zum Auftragen einer Flüssigkeit (2) auf einen Tupfer (48), umfassend
- einen flexiblen Behälter (1) mit der Flüssigkeit (2) und mit einem einen Verschluss (3) aufweisenden ersten Auslass (4),
- ein Aufsatzteil (6, 22, 31) für den Behälter (1) mit einem zweiten Auslass (10), wobei das Aufsatzteil (6, 22, 31) lösbar an dem Behälter (1) befestigt werden kann und wobei die Flüssigkeit (2) bei geöffnetem Verschluss (3) und am Behälter (1) befestigten Aufsatzteil (6, 22, 31) von dem ersten Auslass (4) zu dem zweiten Auslass (10) fließen kann,
**dadurch gekennzeichnet, dass** das Aufsatzteil (6, 22, 31) einen manuellen, zangenartigen Greifmechanismus zum Ergreifen und zum Halten des Tupfers (48) mit einem ersten Greifarm (12, 23, 32) und einem zweiten Greifarm (13, 26, 33) umfasst,
wobei
- sowohl der erste als auch der zweite Greifarm (12, 13, 32, 33) einen Kopfabschnitt (14, 15, 34, 35), einen Gelenkabschnitt (18, 19, 38, 39) und einen Griffabschnitt (20, 21, 40, 41) umfasst, oder
- der erste Greifarm (23) einen Kopfabschnitt (24) umfasst und feststehend mit dem Aufsatzteil (22) verbunden ist, wobei der erste Greifarm (23) an der Verbindungsstelle mit dem Aufsatzteil (22) endet, und der zweite Greifarm (26) einen Kopfabschnitt (27), einen Gelenkabschnitt (29) und einen Griffabschnitt (30) umfasst.

2. Medizinisches System nach Anspruch 1, **dadurch gekennzeichnet, dass** der Behälter (1) im Wesentlichen zylinderförmig ist.

3. Medizinisches System nach Anspruch 1 oder 2, **dadurch gekennzeichnet, dass** das den Behälter (1) bildende Material einen Kunststoff umfasst.

4. Medizinisches System nach einem der vorangehenden Ansprüche, **dadurch gekennzeichnet, dass** der erste Auslass (4) einen Einmalverschluss oder einen Membranverschluss (3) aufweist.

5. Medizinisches System nach einem der vorangehenden Ansprüche, **dadurch gekennzeichnet, dass** der zweite Auslass (10) eine einzige Öffnung (11) aufweist oder mehrere Öffnungen (50a, 50b, 50c, 50d) umfasst.

6. Medizinisches System nach einem der vorangehenden Ansprüche, **dadurch gekennzeichnet, dass** das Aufsatzteil (6, 22, 31) einen Hohlkörper (7) mit einem ersten offenen Ende (8) und einem zweiten offenen Ende (9) umfasst, wobei
- das erste offene Ende (8) zur Befestigung des Aufsatzteils (6, 22, 31) an dem Behälter (1) vorgesehen ist und den ersten Auslass (4) aufnehmen kann,
- das zweite offene Ende (9) den zweiten Auslass (10) bildet, und
- der erste Greifarm (12, 23, 32) und der zweite Greifarm (13, 26, 33) an dem Hohlkörper (7) befestigt sind.

7. Medizinisches System nach Anspruch 6, **dadurch gekennzeichnet, dass** der Hohlkörper (7) im Wesentlichen die Form eines Zylinders oder eines Kegelstumpfes aufweist, wobei
- eine Grundfläche des Zylinders oder des Kegelstumpfes das erste offene Ende (8) umfasst,
- eine Deckfläche des Zylinders oder des Kegelstumpfes das zweite offene Ende (9) umfasst, und
- der erste Greifarm (12, 23, 32) und der zweite Greifarm (13, 26, 33) an einer Mantelfläche des Zylinders oder des Kegelstumpfes befestigt sind.

8. Medizinisches System nach einem der vorangehenden Ansprüche, **dadurch gekennzeichnet,**
- **dass** das Aufsatzteil (6, 22) mindestens ein federndes Element umfasst, so dass sich der Greifmechanismus (12, 13, 23, 26) selbstständig in eine Ausgangsstellung bewegen kann, wenn sich der Greifmechanismus (12, 13, 23, 26) in einem unbetätigten Zustand befindet, und
- **dass** der erste und der zweite Greifarm (12, 13, 23, 26) derart ausgestaltet sind, dass der Greifmechanismus (12, 13, 23, 26) in der Ausgangsstellung im Wesentlichen geschlossen ist und bei Betätigung geöffnet werden kann.

9. Medizinisches System nach einem der Ansprüche 1 bis 7, **dadurch gekennzeichnet,**
- **dass** das Aufsatzteil (31) mindestens ein federndes Element umfasst, so dass sich der Greifmechanismus (32, 33) selbstständig in eine Ausgangsstellung bewegen kann, wenn sich der Greifmechanismus (32, 33) in einem unbetätigten Zustand befindet, und
- **dass** der erste und der zweite Greifarm (32, 33) derart ausgestaltet sind, dass der Greifmechanismus (32, 33) in der Ausgangsstellung geöffnet ist und bei Betätigung geschlossen werden kann.

10. Medizinisches System nach einem der vorangehenden Ansprüche, **dadurch gekennzeichnet, dass** der Behälter (1) oder das Aufsatzteil (6, 22, 31) ein Flatterventil (46) umfasst.

11. Medizinisches System nach einem der vorangehenden Ansprüche, **dadurch gekennzeichnet, dass** das Aufsatzteil (6, 22, 31) lösbar an dem Behälter (1) befestigt werden kann, indem das Aufsatzteil dem Behälter aufgesteckt werden kann oder indem das Aufsatzteil (6, 22, 31) an dem Behälter (1) angeschraubt werden kann.

12. Medizinisches System nach einem der vorangehenden Ansprüche, **dadurch gekennzeichnet, dass** das Aufsatzteil (6) ein Mittel zum Durchstoßen eines Membranverschlusses (42) umfasst.

13. Kit, umfassend
- ein steriles medizinisches System nach einem der Ansprüche 1 bis 12, und
- einen oder mehrere sterile Tupfer (48).

14. Verfahren zum Auftragen einer Flüssigkeit (2) auf einen Tupfer (48), umfassend die Schritte:
i. Bereitstellen eines medizinischen Systems nach einem der Ansprüche 1 bis 12,
ii. Öffnen des Verschlusses (3) des Behälters (1),
iii. Befestigen des Aufsatzteils (6) an dem Behälter (1), um einen Applikator (47) auszubilden,
iv. Bereitstellen des Tupfers (48),
v. Ergreifen und Halten des Tupfers (48) mit dem Greifmechanismus (12, 13),
vi. Auftragen eines Teils der in dem Behälter (1) enthaltenen Flüssigkeit (2) oder der gesamten in dem Behälter (1) enthaltenen Flüssigkeit (2) auf den Tupfer (48), indem der Behälter (1) einmal oder mehrmals zusammengedrückt wird.

## Claims

1. Medical system for applying a liquid (2) to a swab (48), comprising
- a flexible container (1) with the liquid (2) and with a first outlet (4) having a closure (3),
- an attachment part (6, 22, 31) for the container (1), with a second outlet (10), wherein the attachment part (6, 22, 31) can be fastened releasably to the container (1), and wherein, with the closure (3) opened and the attachment part (6, 22, 31) fastened to the container (1), the liquid (2) is able to flow from the first outlet (4) to the second outlet (10),
**characterized in that** the attachment part (6, 22, 31) comprises a manual, tweezer-like gripping mechanism for gripping and holding the swab (48), with a first gripping arm (12, 23, 32) and a second gripping arm (13, 26, 33), wherein
- both the first and the second gripping arm (12, 13, 32, 33) have a head portion (14, 15, 34, 35), a hinge portion (18, 19, 38, 39) and a handle portion (20, 21, 40, 41), or
- the first gripping arm (23) comprises a head portion (24) and is fixedly connected to the attachment part (22), wherein the first gripping arm (23) ends at the junction to the attachment part (22), and the second gripping arm (26) has a head portion (27), a hinge portion (29) and a handle portion (30).

2. Medical system according to Claim 1, **characterized in that** the container (1) is substantially cylindrical.

3. Medical system according to Claim 1 or 2, **characterized in that** the material forming the container (1) comprises a plastic.

4. Medical system according to one of the preceding claims, **characterized in that** the first outlet (4) has a disposable closure or a membrane closure (3).

5. Medical system according to any one of the preceding claims, **characterized in that** the second outlet (10) has a single opening (11) or comprises a plurality of openings (50a, 50b, 50c, 50d).

6. Medical system according to one of the preceding claims, **characterized in that** the attachment part (6, 22, 31) comprises a hollow body (7) with a first open end (8) and a second open end (9), wherein
- the first open end (8) is provided for fastening the attachment part (6, 22, 31) to the container (1) and can receive the first outlet (4),
- the second open end (9) forms the second outlet (10), and
- the first gripping arm (12, 23, 32) and the second gripping arm (13, 26, 33) are fastened on the hollow body (7).

7. Medical system according to Claim 6, **characterized in that** the hollow body (7) has substantially the shape of a cylinder or of a truncated cone, wherein
- a base of the cylinder or of the truncated cone comprises the first open end (8),
- a top of the cylinder or of the truncated cone comprises the second open end (9), and
- the first gripping arm (12, 23, 32) and the second gripping arm (13, 26, 33) are fastened to a lateral surface of the cylinder or of the truncated cone.

8. Medical system according to one of the preceding claims, **characterized in that**
- the attachment part (6, 22) comprises at least one resilient element, such that the gripping mechanism (12, 13, 23, 26) can move autonomously to a starting position when the gripping mechanism (12, 13, 23, 26) is in an unactuated state, and
- the first and the second gripping arm (12, 13, 23, 26) are designed such that the gripping mechanism (12, 13, 23, 26) is substantially closed in the starting position and can be opened when actuated.

9. Medical system according to one of Claims 1 to 7, **characterized in that**
- the attachment part (31) comprises at least one resilient element, such that the gripping mechanism (32, 33) can move autonomously to a starting position when the gripping mechanism (32, 33) is in an unactuated state, and
- the first and the second gripping arm (32, 33) are designed such that the gripping mechanism (32, 33) is open in the starting position and can be closed when actuated.

10. Medical system according to one of the preceding claims, **characterized in that** the container (1) or the attachment part (6, 22, 31) comprises a flutter valve (46).

11. Medical system according to one of the preceding claims, **characterized in that** the attachment part (6, 22, 31) can be fastened releasably to the container (1), by the attachment part being able to be plugged onto the container or by the attachment part (6, 22, 31) being able to be screwed onto the container (1).

12. Medical system according to one of the preceding claims, **characterized in that** the attachment part (6) comprises a means for piercing a membrane closure (42).

13. Kit comprising
- a sterile medical system according to one of Claims 1 to 12, and
- one or more sterile swabs (48).

14. Method for applying a liquid (2) to a swab (48), comprising the steps of:
i. providing a medical system according to one of Claims 1 to 12,
ii. opening the closure (3) of the container (1),
iii. fastening the attachment part (6) to the container (1) in order to form an applicator (47),
iv. providing the swab (48),
v. gripping and holding the swab (48) with the gripping mechanism (12, 13),
vi. applying some of the liquid (2) contained in the container (1) or all of the liquid (2) contained in the container (1) to the swab (48), by compressing the container (1) once or several times.

## Revendications

1. Système médical pour appliquer un liquide (2) sur une compresse (48), comprenant
- un récipient flexible (1) avec le liquide (2) et avec une première sortie (4) présentant une fermeture (3),
- une partie supérieure (6, 22, 31) pour le récipient (1) avec une deuxième sortie (10), la partie supérieure (6, 22, 31) pouvant être fixée de manière amovible sur le récipient (1) et le liquide (2) pouvant s'écouler de la première sortie (4) vers la deuxième sortie (10) lorsque la fermeture (3) est ouverte et que la partie supérieure (6, 22, 31) est fixée sur le récipient (1), **caractérisé en ce que** la partie supérieure (6, 22, 31) comprend un mécanisme de préhension manuel de type pince pour saisir et maintenir la compresse (48), avec un premier bras de préhension (12, 23, 32) et un deuxième bras de préhension (13, 26, 33),
- le premier et le deuxième bras de préhension (12, 13, 32, 33) comprenant tous deux une section de tête (14, 15, 34, 35), une section d'articulation (18, 19, 38, 39) et une section de poignée (20, 21, 40, 41), ou
- le premier bras de préhension (23) comprenant une section de tête (24) et étant relié de manière fixe à la partie supérieure (22), le premier bras de préhension (23) se terminant au niveau de la jonction avec la partie supérieure (22), et le deuxième bras de préhension (26) comprenant une section de tête (27), une section d'articulation (29) et une section de poignée (30).

2. Système médical selon la revendication 1, **caractérisé en ce que** le récipient (1) est essentiellement de forme cylindrique.

3. Système médical selon la revendication 1 ou 2, **caractérisé en ce que** le matériau formant le récipient (1) comprend une matière plastique.

4. Système médical selon l'une quelconque des revendications précédentes, **caractérisé en ce que** la première sortie (4) présente une fermeture à usage unique ou une fermeture à membrane (3).

5. Système médical selon l'une quelconque des revendications précédentes, **caractérisé en ce que** la deuxième sortie (10) présente une seule ouverture (11) ou comprend plusieurs ouvertures (50a, 50b, 50c, 50d).

6. Système médical selon l'une quelconque des revendications précédentes, **caractérisé en ce que** la partie supérieure (6, 22, 31) comprend un corps creux (7) avec une première extrémité ouverte (8) et une deuxième extrémité ouverte (9),
- la première extrémité ouverte (8) pour fixer la partie supérieure (6, 22, 31) étant prévue sur le récipient (1) et pouvant recevoir la première sortie (4),
- la deuxième extrémité ouverte (9) formant la deuxième sortie (10), et
- le premier bras de préhension (12, 23, 32) et le deuxième bras de préhension (13, 26, 33) étant fixés au corps creux (7).

7. Système médical selon la revendication 6, **caractérisé en ce que** le corps creux (7) a essentiellement la forme d'un cylindre ou d'un cône tronqué,
- une surface de base du cylindre ou du cône tronqué comprenant la première extrémité ouverte (8),
- une surface de recouvrement du cylindre ou du cône tronqué comprenant la deuxième extrémité ouverte (9), et
- le premier bras de préhension (12, 23, 32) et le deuxième bras de préhension (13, 26, 33) étant fixés à une surface d'enveloppe du cylindre ou du cône tronqué.

8. Système médical selon l'une quelconque des revendications précédentes, **caractérisé en ce que**
- la partie supérieure (6, 22) comprend au moins un élément élastique, de telle sorte que le mécanisme de préhension (12, 13, 23, 26) peut se déplacer de manière autonome dans une position initiale lorsque le mécanisme de préhension (12, 13, 23, 26) se trouve dans un état non actionné, et
- le premier et le deuxième bras de préhension (12, 13, 23, 26) sont conçus de telle sorte que le mécanisme de préhension (12, 13, 23, 26) est essentiellement fermé dans la position initiale et peut être ouvert lors de l'actionnement.

9. Système médical selon l'une quelconque des revendications 1 à 7, **caractérisé en ce que**
- la partie supérieure (31) comprend au moins un élément élastique, de telle sorte que le mécanisme de préhension (32, 33) peut se déplacer de manière autonome dans une position initiale lorsque le mécanisme de préhension (32, 33) se trouve dans un état non actionné, et
- le premier et le deuxième bras de préhension (32, 33) sont conçus de telle sorte que le mécanisme de préhension (32, 33) est ouvert dans la position initiale et peut être fermé lors de l'actionnement.

10. Système médical selon l'une quelconque des revendications précédentes, **caractérisé en ce que** le récipient (1) ou la partie supérieure (6, 22, 31) comprend une soupape flexible (46).

11. Système médical selon l'une quelconque des revendications précédentes, **caractérisé en ce que** la partie supérieure (6, 22, 31) peut être fixée de manière amovible sur le récipient (1), par le fait que la partie supérieure peut être enfichée sur le récipient ou par le fait que la partie supérieure (6, 22, 31) peut être vissée sur le récipient (1).

12. Système médical selon l'une quelconque des revendications précédentes, **caractérisé en ce que** la partie supérieure (6) comprend un moyen pour percer une fermeture à membrane (42).

13. Kit, comprenant
- un système médical stérile selon l'une quelconque des revendications 1 à 12, et
- une ou plusieurs compresses stériles (48).

14. Procédé d'application d'un liquide (2) sur une compresse (48), comprenant les étapes suivantes :
i. la fourniture d'un système médical selon l'une quelconque des revendications 1 à 12,
ii. l'ouverture de la fermeture (3) du récipient (1),
iii. la fixation de la partie supérieure (6) au récipient (1) afin de former un applicateur (47),
iv. la fourniture de la compresse (48),
v. la saisie et le maintien de la compresse (48) avec le mécanisme de préhension (12, 13),
vi. l'application d'une partie du liquide (2) contenu dans le récipient (1) ou de la totalité du liquide (2) contenu dans le récipient (1) sur la compresse (48) en comprimant le récipient (1) une fois ou plusieurs fois.
